# FASCICULE DE BREVET EUROPEEN

(11) **EP 2 646 544 B1**
(45) Date de publication et mention de la délivrance du brevet: **02.11.2016**
(21) Numéro de dépôt: 11794083.3
(22) Date de dépôt: 02.12.2011
(51) Int. Cl.: C12N 9/04, C12N 9/92

(54) **PROCÉDÉ DE PREPARATION D'UNE LEVURE INDUSTRIELLE, LEVURE INDUSTRIELLE ET APPLICATION À LA PRODUCTION D'ETHANOL A PARTIR D'AU MOINS UN PENTOSE**
VERFAHREN ZUR HERSTELLUNG VON INDUSTRIELLER HEFE, INDUSTRIELLE HEFE UND IHRE VERWENDUNG ZUR HERSTELLUNG VON ETHANOL AUS MINDESTENS EINER PENTOSE
METHOD FOR PREPARING AN INDUSTRIAL YEAST, INDUSTRIAL YEAST AND USE IN THE PRODUCTION OF ETHANOL FROM AT LEAST ONE PENTOSE

(30) Priorité: 03.12.2010 FR 1004709
(43) Date de publication de la demande: 09.10.2013
(73) Titulaire: Lesaffre et Compagnie, 75001 Paris (FR)
(72) Inventeur: DESFOUGERES, Thomas, 59960 Neuville en Ferrain (FR); PIGNEDE, Georges, 59700 Marcq en Baroeul (FR)
(74) Mandataire: Cabinet Plasseraud
(86) Numéro de dépôt international: PCT/EP2011/071616
(87) Numéro de publication internationale: WO 2012/072793

(56) Documents cités:
- WO-A1-2009/109634
- US-A1- 2009 246 844
- US-A1- 2010 291 648
- VAN MARIS A J A ET AL: "Development of Efficient Xylose Fermentation in Saccharomyces cerevisiae: Xylose Isomerase as a Key Component", ADVANCES IN BIOCHEMICAL ENGINEERING, BIOTECHNOLOGY, SPRINGER, BERLIN, DE, vol. 108, 1 janvier 2007 (2007-01-01), pages 179-204, XP008086128, ISSN: 0724-6145
- KARHUMAA KAISA ET AL: "Investigation of limiting metabolic steps in the utilization of xylose by recombinant Saccharomyces cerevisiae using metabolic engineering", 15 avril 2005 (2005-04-15), YEAST, JOHN WILEY & SONS LTD, GB, PAGE(S) 359 - 368, XP002494168, ISSN: 0749-503X abrégé
- BETTIGA MAURIZIO ET AL: "Comparing the xylose reductase/xylitol dehydrogenase and xylose isomerase pathways in arabinose and xylose fermenting Saccharomyces cerevisiae strains", BIOTECHNOLOGY FOR BIOFUELS, BIOMED CENTRAL LTD, LONDON UK, vol. 1, no. 1, 23 octobre 2008 (2008-10-23), page 16, XP021045780, ISSN: 1754-6834, DOI: DOI:10.1186/1754-6834-1-16
- AKINORI MATSUSHIKA ET AL: "Ethanol production from xylose in engineered Saccharomyces cerevisiae strains: current state and perspectives", APPLIED MICROBIOLOGY AND BIOTECHNOLOGY, SPRINGER, BERLIN, DE, vol. 84, no. 1, 2 juillet 2009 (2009-07-02), pages 37-53, XP019737770, ISSN: 1432-0614, DOI: DOI:10.1007/S00253-009-2101-X
- BENGTSSON O ET AL: "Xylose reductase from Pichia stipitis with altered coenzyme preference improves ethanolic xylose fermentation by recombinant Saccharomyces cerevisiae", BIOTECHNOLOGY FOR BIOFUELS, BIOMED CENTRAL LTD, LONDON UK, vol. 2, 5 mai 2009 (2009-05-05), pages 1-10, XP002612933, ISSN: 1754-6834, DOI: DOI:10.1186/1754-6834-2-9
- BÄRBEL HAHN-HÄGERDAL ET AL: "Towards industrial pentose-fermenting yeast strains", APPLIED MICROBIOLOGY AND BIOTECHNOLOGY, SPRINGER, BERLIN, DE, vol. 74, no. 5, 9 février 2007 (2007-02-09), pages 937-953, XP019489564, ISSN: 1432-0614, DOI: DOI:10.1007/S00253-006-0827-2

## Description

### DOMAINE TECHNIQUE

Le présent exposé se rapporte au domaine des procédés d'obtention de souches de levure productrices d'éthanol, des levures ainsi produites, et de la production industrielle d'éthanol à partir desdites levures. Plus spécialement le présent exposé concerne dans son aspect le plus général, un procédé de préparation de levures à partir de souches de *Saccharomyces cerevisiae* dites industrielles, lesdites levures et leur application à la production industrielle d'éthanol à partir de milieux industriels contenant au moins un pentose, notamment le xylose.

### ETAT DE LA TECHNIQUE

Le point commun de la plupart des approches de l'art antérieur du domaine consiste en des procédés visant l'amélioration de souches au patrimoine génétique connu et/ou construit et dont les aptitudes à produire de l'éthanol sont étudiées en général dans des milieux et dans des conditions « idéales » de laboratoire.

En effet, la littérature scientifique ainsi que les documents brevet analysés par la Demanderesse enseignent, le plus souvent, des procédés d'obtention de souches haploïdes ou diploïdes, faiblement tolérantes aux stress notamment aux fortes concentrations d'éthanol et/ou aux températures élevées et/ou aux inhibiteurs de fermentation. En outre, ces procédés nécessitent pour la plupart d'avoir recours, pour ces souches, à l'utilisation de marqueurs d'auxotrophie et/ou de marqueurs de résistance à des antibiotiques qui peuvent les disqualifier pour une utilisation ultérieure en milieu industriel pour des raisons évidentes de coût voire quelquefois de santé ou de respect de l'environnement.

Les propriétés de croissance des souches développées antérieurement sont en général insuffisantes et ces souches n'ont jamais été confrontées aux impératifs de production de biomasse à l'échelle industrielle, à savoir pour n'en citer que trois : fort taux de croissance, aptitude au séchage, stabilité en conservation.

Si des performances dites fermentaires (aptitude à la production anaérobie d'éthanol) sont obtenues dans des milieux synthétiques ou définis dits de laboratoire avec ces souches antérieures, elles ne sont pas transposables en général, dans des milieux industriels comportant des mélanges complexes issus par exemple de résidus de traitement de la cellulose qui renferment des composés toxiques pouvant inhiber à différents niveaux la machinerie cellulaire de la levure, notamment furfural, HMF, dérivés phénoliques, acide acétique. En outre, l'aptitude au « scale up » ou transposition d'échelle de ces procédés antérieurs de production d'éthanol est rarement documentée.

Le document WO 2008/133665 enseigne la production d'alcool à partir d'une souche de levure au « contexte génétique » de type :
- Gène STP15 muté (F117S, Y195H, K218R).
- Gènes codant pour XI / XR / XDH ou XK exogènes.

« XI » désigne la xylose isomérase, « XR » désigne la xylose réductase, « XDH » désigne la xylitol déshydrogénase, et « XK » désigne la D-xylulokinase.

Le document WO 2005/113774 décrit un opéron recombinant comprenant deux séquences d'acide nucléique codant respectivement une XI d'*E*. *coli* et une XDH de *Trichoderma reesei* dans le cadre de la production de xylitol.

Le document Plos Genetics de Gavin Sherlok et al., publié le 13 mai 2010, décrit un gène *XDH1* présent dans certaines souches particulières de *Saccharomyces cerevisiae,* codant possiblement une xylitol déshydrogénase.

Le document au nom de Dawid Brat, Eckard Boles et Beate Wiedemannn, dans Appl. Environ. Microbiol., avril 2009, vol 75, n°8, p. 2304-2311, décrit l'expression chez *Saccharomyces cerevisiae* du gène de la xylose isomérase issu de *Clostridium phytofermentans.*

Il ressort de cette revue des documents de l'art antérieur, ainsi que des travaux des inventeurs de la présente invention, qu'étant donné les contextes/patrimoines génétiques très différents des souches de levures *Saccharomyces cerevisiae* mises en oeuvre dans le but de croître sur et/ou fermenter le xylose, les conséquences par exemple, d'une surexpression et/ou d'une délétion de gènes natifs et/ou de l'introduction d'un ou de plusieurs gènes hétérologues ne se peuvent prédire.

### RESUME DE L'INVENTION

Aussi, La Demanderesse étudiant de nombreuses souches de type alcool, brassicoles et de boulangerie, a constaté de manière surprenante que l'introduction dans des souches de levure de cassettes d'expression ou de délétion déterminées de sorte à y exprimer une voie métabolique XI-XDH, rendait celles-ci particulièrement performantes dans la production d'éthanol. De plus, la demanderesse a constaté que l'introduction de l'acide nucléique codant XI n'est pas suffisante à elle seule pour la fermentation efficace du Xylose.

De manière générale, la demanderesse a constaté que l'introduction de cassettes d'expression d'un gène codant pour une enzyme apte à transformer tout hydrate de carbone (notamment D-xylose) en xylulose (D-xylulose) et d'un gène codant pour une enzyme apte à transformer tout pentol (notamment xylitol) en xylulose en une seule étape, rendait toutes souches ainsi modifiées particulièrement performantes dans la croissance sur et/ou la fermentation du xylose.

Par « enzyme apte à transformer le xylose en xylulose », on entend une enzyme xylose isomérase.

Par « enzyme apte à transformer en une seule étape le xylitol en xylulose », on entend une enzyme xylitol déshydrogénase.

En effet, la demanderesse a confirmé que contrairement à la voie dite fongique associant XR et XDH, la voie dite bactérienne d'isomérisation (dont un exemple est celle de *C. phytofermentans*), quand elle est mise en oeuvre, n'implique pas de co-substrats. De plus, cette voie permet d'éviter l'accumulation de xylitol qui est un intermédiaire métabolique présent dans la voie fongique et qui peut réduire de manière significative le rendement de production d'éthanol.

Très récemment, sur certaines souches de S. *cerevisiae,* notamment celles utilisés pour la vinification, un gène *XDH1* a été identifié comme étant essentiel pour le métabolisme du xylose desdites souches (PLoS genetics 2010, 6, 1-17). Aussi, la demanderesse a constaté que même en supprimant le gène *GRE3* des souches modifiées, il existe d'autres activités parasites qui peuvent transformer le xylose en xylitol (activité aldose réductase). Ce qui est néfaste pour l'activité XI réduisant ainsi le rendement en éthanol recherché.

Les travaux réalisés par la demanderesse montrent que le renforcement de l'activité Xylitol DésHydrogénase se traduit par l'absence d'inhibition de la XI et donc par la stimulation de cette voie. Ce qui permet la production d'éthanol, à partir d'un milieu comportant au moins du xylose, avec un bon rendement cinétique.

En d'autres termes, dans l'état de la technique, deux voies différentes ont été explorées pour permettre la fermentation du xylose par la levure : la voie dite fongique utilisant les enzymes XR et XDH ; et la voie dite bactérienne utilisant l'enzyme XI. L'objet du présent exposé combine d'une manière originale des enzymes issues de ces deux voies, afin d'obtenir un résultat amélioré.

L'invention est définie dans les revendications en annexe.

L'exposé concerne en particulier une souche de levure comportant au moins une copie d'un gène exogène codant pour une xylose isomérase, et une copie d'un gène exogène codant pour une xylitol déshydrogénase.

Par gène « *exogène* » (par opposition à « *endogène* »), on entend un gène qui n'est pas présent naturellement dans l'espèce de levure considérée. Le gène codant pour une xylitol déshydrogénase peut être le gène *XYL2,* mais dans ce cas il s'agit du gène *XYL2* d'une autre espèce que celle de la souche considérée.

L'exposé concerne également un procédé de préparation d'une souche de levure comportant au moins une copie d'un gène exogène codant pour une xylose isomérase, et une copie d'un gène exogène codant pour une xylitol déshydrogénase.

L'exposé concerne également un procédé de production d'éthanol utilisant les souches de levure selon l'invention.

### BREVE DESCRIPTION DES FIGURES

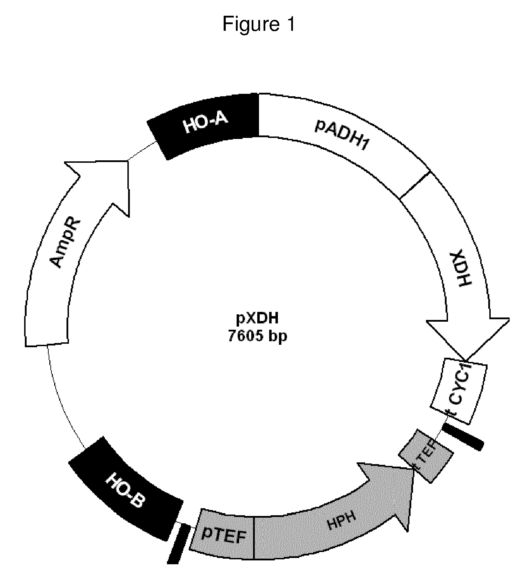

- La Figure 1 illustre un vecteur de surexpression de la XDH de *Pichia stipitis.*

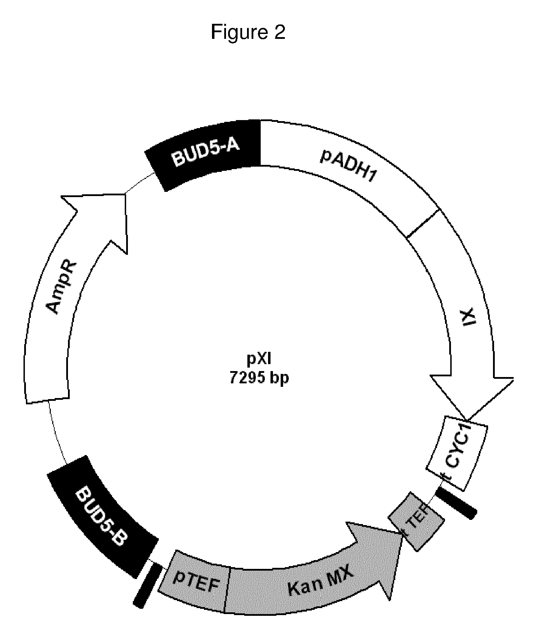

- La Figure 2 illustre un vecteur de surexpression de la XI de *Clostridium phytofermentans.*

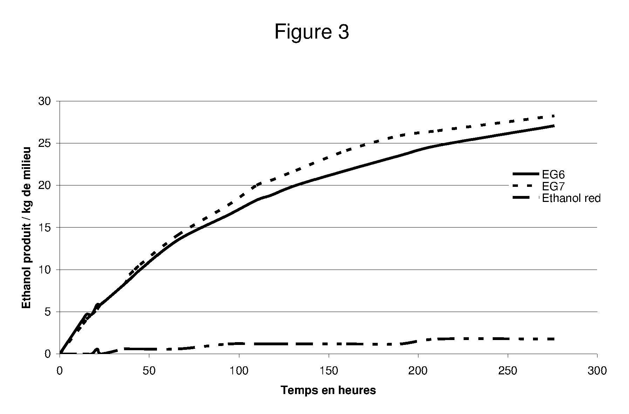

- La Figure 3 est un graphe illustrant la production d'éthanol en fonction du temps de fermentation à 32°C de deux souches de levure conformes à l'invention après évolution dirigée et de la souche éthanol red ™. Les clones testés ont été inoculés à raison de 5g de masse sèche/L dans un milieu YF + xylose à 70 g/L.

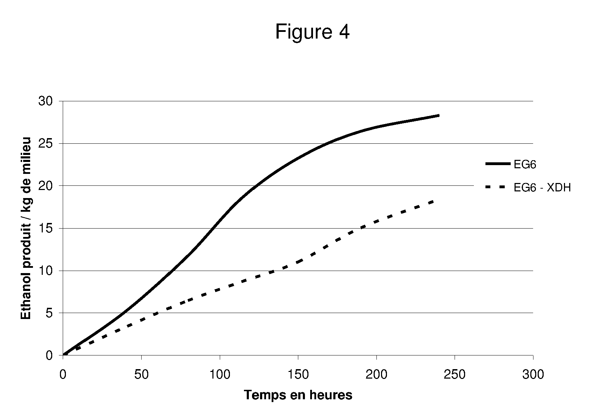

- La Figure 4 est un graphe illustrant la production d'éthanol en fonction du temps de fermentation à 32°C de deux souches de levure, l'une conforme à l'invention après évolution dirigée, l'autre souche découlant de la première mais après substitution de la copie du gène XDH par un marqueur de résistance à la kanamycine (KanMX4). Les clones testés ont été inoculés à raison de 5g de masse sèche/L dans un milieu YF + xylose à 70g/L.

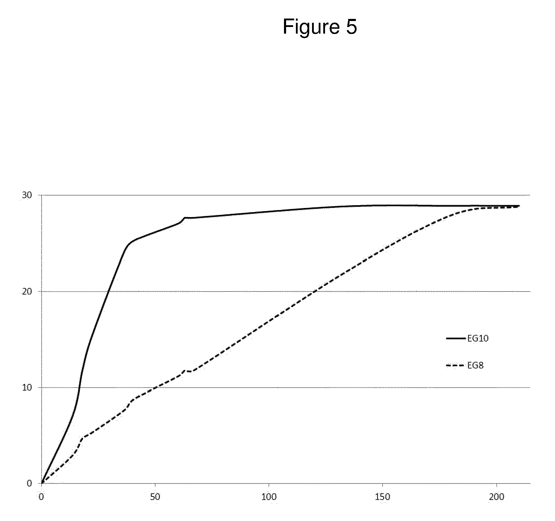

- La figure 5 est un graphe illustrant la production d'éthanol (en ordonnée, en g pour 1 kg de milieu) en fonction du temps de fermentation à 32°C (en abscisse, en heures) de deux souches le levure conformes à l'invention EG8 et EG10. Les clones testés ont été inoculés à raison de 0,25 g de matière sèche levure par kg de milieu YF contenant du xylose à 70g/l comme seule source de carbone.

### DESCRIPTION DETAILLEE DE MODES DE REALISATION

Ainsi, le présent exposé a pour premier objet un procédé de préparation d'une souche de levure *Saccharomyces cerevisiae* apte à produire de l'éthanol à partir d'un milieu comportant au moins un pentose (notamment le xylose) et qui comprend les étapes suivantes consistant à
(i) sélectionner (ou fournir) une souche de levure *Saccharomyces cerevisiae*
(ii) intégrer les cassettes d'expression suivantes dans le génome de la levure de l'étape (i),
   a. l'association du type cadre ouvert de lecture (ORF) d'un gène codant pour une enzyme apte à transformer tout hydrate de carbone, notamment le xylose, en xylulose sous la dépendance d'un promoteur et d'un terminateur de *Saccharomyces cerevisiae,* la dite cassette étant flanquée en amont et en aval de régions recombinogéniques permettant son intégration ciblée dans le génome,
   b. l'association du type cadre ouvert de lecture (ORF) d'un gène codant pour une enzyme apte à transformer en une seule étape tout pentol, notamment le xylitol, en xylulose sous la dépendance d'un promoteur et d'un terminateur de *Saccharomyces cerevisiae,* la dite cassette étant flanquée en amont et en aval de régions recombinogéniques permettant son intégration ciblée dans le génome,
(iii) induire l'expression d'au moins un gène de chaque étape de la partie non oxydative de la voie des pentoses phosphate ainsi qu'au moins un gène codant la xylulokinase (XKS1) en les plaçant sous la dépendance d'un promoteur d'un gène, notamment de la glycolyse, non réprimé ni par l'anaérobiose ni par la répression catabolique et fortement exprimé lors d'une fermentation alcoolique, et
(iv) déléter au moins au moins une copie ou de préférence au moins deux copies du cadre ouvert de lecture (ORF) du gène de *Saccharomyces cerevisiae GRE3* codant pour une aldose réductase. De préférence la totalité des copies du cadre ouvert de lecture (ORF) du gène de *Saccharomyces cerevisiae GRE3* sont délétées.

Le gène *XKS1* est de préférence le gène figurant dans GenBank sous le numéro 853108.

Le gène *GRE3* est de préférence le gène figurant dans GenBank sous le numéro 856504.

De manière préférentielle, le gène de l'étape (ii)a est un gène XI codant pour l'enzyme xylose isomérase choisi parmi ceux présents dans les génomes des genres *Clostridium, Pyromyces, Bactéroïdes, Streptomyces, Haemophilus, Burkholderia, Enterococcus, Thermotoga, Fusobacterium, Geobacillus, Arthrobacter, Ciona, Physcomitrella, Cellvibrio, Chitinophaga, Saccharopolyspora, Salinibacter.*

Le gène XI est de préférence choisi parmi un gène de *Clostridium phytofermentans, Saccharopolyspora erythraea, Salinibacter ruber* ou de *Piromyces sp. E2.*

Selon un mode de réalisation préféré, le gène XI a pour séquence la séquence de nucléotides SEQ ID NO:1 (qui représente la séquence du gène XI de *Clostridium phytofermentans,* décrite dans le document DE 102008031350). Alternativement, le gène XI a une séquence présentant au moins 70 % d'identité, de préférence au moins 75 % d'identité, ou au moins 80 % d'identité, ou au moins 85 % d'identité, ou au moins 90 % d'identité, ou au moins 95 % d'identité, ou au moins 98 % d'identité, ou au moins 99 % d'identité, avec la séquence SEQ ID NO:1, et il code une enzyme xylose isomérase fonctionnelle.

Selon un autre mode de réalisation, le gène XI présente une séquence codant pour un polypeptide ayant la séquence d'acides aminés SEQ ID NO:2 (qui représente la séquence de la protéine XI de *Clostridium phytofermentans,* décrite dans le document DE 102008031350). Alternativement, ledit polypeptide a une séquence présentant au moins 70 % d'identité, de préférence au moins 75 % d'identité, ou au moins 80 % d'identité, ou au moins 85 % d'identité, ou au moins 90 % d'identité, ou au moins 95 % d'identité, ou au moins 98 % d'identité, ou au moins 99 % d'identité, avec la séquence SEQ ID NO:2, et il a une activité xylose isomérase.

Selon la présente invention par « transformer le xylitol en xylulose en une seule étape » on entend une oxydation directe du xylitol en xylulose et cela par une seule et même enzyme (xylitol déshydrogénase).

Préférentiellement le gène de l'étape (ii)b, est un gène de *Pichia stipitis* codant pour l'enzyme xylitol déshydrogénase XDH.

De préférence, il s'agit du gène *XYL2,* dont la séquence figure dans GenBank sous le numéro 4852013, ou une séquence présentant au moins 70 % d'identité, de préférence au moins 75 % d'identité, ou au moins 80 % d'identité, ou au moins 85 % d'identité, ou au moins 90 % d'identité, ou au moins 95 % d'identité, ou au moins 98 % d'identité, ou au moins 99 % d'identité, par rapport à ladite séquence figurant dans GenBank sous le numéro 4852013, la séquence codant pour une enzyme xylitol déshydrogénase fonctionnelle.

De manière préférentielle, la souche de levure de l'étape (i) présente une activité xylitol déshydrogénase XDH endogène inférieure à 150 mKat / g de protéines. L'activité xylitol déshydrogénase peut être mesurée dans les conditions indiquées dans l'article de Xu et al., intitulé Characterization of Ethanol Production from Xylose and Xylitol by a Cell-Free Pachysolen tannophilus System, dans Appl. Environ. Microbiol. 59:231-235 (1993).

Il est connu que, lors du traitement de la biomasse destinée à la fermentation alcoolique, certains inhibiteurs de fermentation apparaissent. Parmi eux on peut citer les produits phénoliques, le furfural ou encore l'acide acétique. Il est connu aussi que ces inhibiteurs sont néfastes pour les performances voire la survie de la levure.

Pour résoudre ce problème additionnel, la demanderesse propose de sélectionner la souche de l'étape i parmi les souches industrielles présentant une résistance aux dérivés phénoliques.

Un autre avantage de la voie XI 'est la possibilité de « greffer en parallèle la voie arabinose bactérienne » comme décrit par exemple dans EP 1 499 708 ou encore dans WO 2008/041840, cette combinaison permettant alors d'augmenter le degré d'alcool final en cas de présence d'arabinose dans les milieux à fermenter.

Le procédé de préparation de la levure de la présente invention tient compte à la fois des contraintes du levurier et dans le même temps de celles d'un utilisateur final dans ses applications notamment en termes de production industrielle d'éthanol à faible coût et haut rendement.

Le procédé selon l'invention présente, en particulier, les avantages suivants :
Pour le levurier, il permet :
   - la construction d'une souche de levure *Saccharomyces cerevisiae* aneu/polyploïde, prototrophe afin de permettre la production de biomasse sur des sources simples de carbone, azote, phosphore dans des milieux peu chers tels que les sous-produits de l'industrie sucrière comme la mélasse par exemple,
   - de disposer d'une souche de levure *Saccharomyces cerevisiae* présentant un taux de croissance maximum (µ max) compris entre 0,37 h⁻¹ et 0,5 h⁻¹,
   - de disposer d'une souche de levure *Saccharomyces cerevisiae* qui, lorsqu'elle est produite selon un procédé tel que décrit dans le livre de référence « Yeast Technology » (2ème édition, 1991, G. Reed et T.W. Nagodawithana, publié par Van Nostrand Reinhold , ISBN 0-442-31892-8), permet d'obtenir un rendement de production de biomasse d'au moins 45g de matières sèches levure pour 100g d'équivalent saccharose mis en oeuvre,
   - de disposer d'une souche de levure *Saccharomyces cerevisiae* résistante au procédé de séchage tel que décrit dans les documents brevets EP 511108 et US 5 741 695, la perte d'activité fermentative après séchage ne devant pas excéder 30%.
   - la production en conditions industrielles (en particulier milieu peu cher, bon rendement de biomasse, levure sèche prête à l'emploi) d'une levure fraîche ou sèche à partir d'une souche de levure *Saccharomyces cerevisiae* génétiquement stable, robuste car notamment tolérante aux concentrations élevées d'éthanol et apte à produire, à partir par exemple de biomasses hémi-cellulosiques, de l'éthanol à au moins 40g/L et ce à une température élevée de l'ordre de 30 à 40°C.

Une souche de levure prototrophe est une souche capable de croître sur un milieu minimum. En particulier, une souche de levure prototrophe selon l'invention est capable de synthétiser tous les acides aminés et bases nécessaires à sa croissance.

Un milieu minimum est un milieu comprenant une source de carbone, une source d'azote, une source de potassium, une source de phosphore, une source de soufre, une source de magnésium, une source de calcium, une source de fer, une source d'oligo-éléments et de l'eau.

Un exemple de milieu minimum est le milieu YNB (Yeast Nitrogen Base). Le milieu YNB comprend par litre : biotine 2 µg, pantothénate de calcium 400 µg, acide folique 2 µg, inositol 2000 µg, niacine 400 µg, acide p-aminobenzoïque 200 µg, hydrochlorure de pyridoxine 400 µg, riboflavine 200 µg, hydrochlorure de thiamine 400 µg, acid borique 500 µg, sulfate de cuivre 40 µg, iodure de potassium 100 µg, chlorure ferrique 200 µg, sulfate de manganèse 400 µg, molybdate de sodium 200 µg, sulfate de zinc 400 µg, phosphate de potassium monobasique 1 g, sulfate de magnésium 500 mg, chlorure de sodium 100 mg, chlorure de calcium 100 mg, sulfate d'ammonium 5g/l, pH final 5,4.

Selon une autre variante préférée du procédé selon l'exposé lorsqu'à l'étape (ii) la cassette d'expression consiste en l'association du type cadre ouvert de lecture (ORF) du gène XI codant pour l'enzyme xylose isomérase *de Clostridium phytofermentans* / promoteur et terminateur de *Saccharomyces cerevisiae,* la dite cassette étant flanquée en amont et en aval de régions recombinogéniques permettant son intégration ciblée dans le génome, ledit procédé comporte alors en outre une étape de Saccharification et de Fermentation Simultanée (SSF) en présence de polymères d'hexoses, majoritairement constitués de glucose, et d'au moins une enzyme apte à les hydrolyser.

Par ailleurs, pour le producteur d'éthanol, l'avantage du procédé selon l'invention est encore de disposer d'une levure active (fraîche - liquide ou comprimée, compressée ou sèche), obtenue selon un procédé de production tel que décrit dans l'ouvrage « Yeast Technology », à partir d'une souche de levure *Saccharomyces cerevisiae* telle que définie au paragraphe précédent qui est :
- capable, dans les conditions de SSF décrites dans le document brevet WO 2004/046333, de fermenter à 32°C un hydrolysat de céréales jusqu'à une concentration en éthanol de 16% (p/p) minimum ;
- capable, dans les conditions de SSF décrites dans le document brevet WO 2004/046333, de fermenter à 35°C un hydrolysat de céréales jusqu'à une concentration en éthanol de 14,5% (p/p) minimum.

Les résultats du procédé selon l'invention sont d'autant plus remarquables lorsqu'ils sont obtenus à partir d'une souche dite industrielle, aneu/polyploïde prototrophe et de fait ayant un matériel génétique nettement plus complexe que celui d'une souche dite de laboratoire, rendant pour le moins imprévisibles les conséquences de modifications de ladite souche industrielle. Ce fond génétique complexe, spécifique aux souches industrielles, rend d'autant plus difficile l'obtention de souches génétiquement modifiées exemptes au final de marqueurs de résistance à des antibiotiques, en particulier lorsque de nombreuses cibles génétiques doivent être modifiées. Des souches exemptes de marqueurs de résistance aux antibiotiques sont de toute évidence préférables pour des raisons de santé et d'environnement.

Les souches prototrophes selon l'invention présentent l'avantage de croître sur des sources simples de carbone, d'azote et de phosphore.

Mais cette caractéristique fait que les vecteurs de transformation disponibles dans la communauté scientifique (vecteurs utilisant des marqueurs d'auxotrophie) sont inopérants.

Il est donc nécessaire d'avoir à disposition des outils/vecteurs utilisant des marqueurs de résistance à des antibiotiques, ces dits outils/marqueurs étant avantageusement construits pour permettre *in fine* l'excision de ces marqueurs. A titre d'exemple, on peut utiliser la technologie Cre-lox. En résumé, on prévoit des séquences loxP encadrant chaque marqueur de sélection. L'excision des marqueurs de sélection est effectuée par transformation de la souche de levure par la méthode de l'acétate de lithium (Schiestl et Gietz, 1989, Current Genetics, vol 16, p339-346), avec un plasmide comprenant le gène de la recombinase Cre et un marqueur de sélection différent du ou des marqueurs de sélection à exciser. L'expression de la recombinase Cre dans la souche de levure permet d'exciser le marqueur de sélection, en ne laissant qu'une séquence loxP, éventuellement avec ses régions flanquantes. Il est possible d'induire ensuite la perte du plasmide comprenant le gène Cre par culture en condition non sélective, c'est-à-dire sur milieu enrichi et en l'absence d'antibiotique. La construction des levures conformes à l'invention a nécessité par exemple l'emploi de 4 marqueurs positifs différents conférant des résistances à 5 antibiotiques différents (généticine, phléomycine, hygromycine, blasticidine et nourseothricine).

Les souches conformes à l'invention sont de préférence aneuploïdes ou polyploïdies : c'est une caractéristique généralement rencontrée dans les levures industrielles qui sont issues du milieu naturel. Le passé phylogénétique de ces souches est à l'origine de cette particularité.

Mais c'est une difficulté supplémentaire rencontrée lorsque l'on souhaite disrupter/inactiver toutes les copies d'un gène donné. Toutefois, ce caractère d'aneu/polyploïdie est en général à l'origine de beaucoup de propriétés d'intérêt des levures industrielles (taux de croissance, résistance à différents stress, stabilité phénotypique).

En outre, la demanderesse après de longues recherches a constaté avec surprise qu'avec le procédé selon l'invention, mis en oeuvre à partir de la souche sélectionnée :
- l'introduction de cassettes d'expression et de délétion ne fragilisait aucunement la levure modifiée qui voit son patrimoine génétique amélioré.

En particulier, les inventeurs ont montré qu'avec ladite souche il est possible de réaliser :
- la délétion d'au moins deux copies du gène *GRE3* de S. *cerevisiae* (l'enzyme Gre3p étant une aldose réductase qui consomme du NADPH,H+ qui est produit en grande partie via la partie oxydative de la voie des pentoses) dans ladite souche industrielle selon l'invention a permis de réduire d'autant la consommation de NADPH,H+ par la dite enzyme,

En variante préférée, ledit au moins un gène de chaque étape de la partie non oxydative de la voie des pentoses phosphate de l'étape (iii) est choisi dans le groupe constitué par les gènes codant pour les enzymes D-ribulose-5-phosphate 3-épimerase, ribose-5-phosphate kétol-isomérase, transkétolase et transaldolase, et notamment dans le groupe constitué par les gènes *RPE1, RKI1, TKL1* et *TAL1.* De préférence, ledit promoteur d'un gène de la glycolyse fortement exprimé lors d'une fermentation alcoolique est le promoteur *TDH3* pour *RPE1, RKI1* et *TKL1,* et *PGK1* pour *TAL1.*

Le gène *TAL1* est de préférence le gène figurant dans GenBank sous le numéro 851068.

Le gène *TKL1* est de préférence le gène figurant dans GenBank sous le numéro 856188.

Le gène *RKI1* est de préférence le gène figurant dans GenBank sous le numéro 854262.

Le gène *RPE1* est de préférence le gène figurant dans GenBank sous le numéro 853322.

Selon des caractéristiques complémentaires ou alternatives, dans le procédé de préparation d'une souche de levure *Saccharomyces cerevisiae* conforme à l'invention :
- le promoteur de *Saccharomyces cerevisiae* des étapes (ii)(a) et (ii)(b) est choisi dans le groupe comprenant les promoteurs des gènes codant les enzymes de la glycolyse et ceux codant pour les enzymes alcool déshydrogénase et de préférence choisi dans le groupe constitué par *ADH1, ADH2, PGK1, TDH3, PDC2* et GAL1/10, de préférence *ADH1.* Le terminateur de *Saccharomyces cerevisiae* est constitué par *CYC1* ou par le terminateur propre du gène de la voie non oxydative des pentoses phosphates.

Il est prévu de préférence une étape ultérieure d'évolution dirigée comportant les étapes successives suivantes consistant à soumettre la levure obtenue à
(i) une mutagenèse,
(ii) une croissance en cultures cycliques sous O₂ limité dans un milieu comportant ledit au moins un pentose, et
(iii) une sélection par croissance aérobie sur milieu solide contenant comme seule source de carbone du glycérol,
   de sorte à procurer des mutants non-déficients respiratoires de ladite levure qui présentent une croissance en anaérobiose en présence d'un milieu comportant ledit au moins un pentose (notamment xylose).

De manière préférée dans cette variante, la mutagenèse de l'étape (i) est réalisée en conditions « douces », à savoir mutagenèse modérée avec 100 à 500J/cm² et, de préférence encore, 300J/cm² de rayonnement UV à 254nm. Ces conditions n'entrainent qu'une mortalité de 7% à 16% de la population soumise au rayonnement UV.

Les inventeurs ont ainsi montré de manière surprenante, qu'avec une si faible mortalité contrôlée, il est permis de réduire d'un facteur 10 la durée de l'étape d'évolution dirigée par cultures cycliques nécessaire à l'obtention de mutants capables de fermenter ledit au moins un pentose (notamment xylose). Le taux de survie est déterminé en étalant sur des boîtes de milieu dont la source de carbone est du glucose, un volume identique de la suspension cellulaire avant et après mutagenèse. Le nombre de colonies est déterminé après 48h de croissance.

De manière préférée, la limitation en O₂ de l'étape (ii) de cette variante est réalisée grâce à une surpression partielle dans l'équipement utilisé (par exemple, fioles ou fermenteurs) due à une surpression consécutive à la production de CO₂ produit.

Les cultures cycliques selon cette variante, dans les conditions de fermentation, permettent d'enrichir la population en mutants capables de fermenter ledit pentose (notamment xylose) et cela en un temps de 2 à 6 semaines et de préférence de 3 à 4 semaines ce qui est relativement court et très intéressant comparé à ce qui serait obtenu par chémostat comme décrit par Kuyper et al. (2004), FEMS Yeast Res., 4, 655-664.

Bien que le phénotype déficient respiratoire « petite » puisse concorder avec les critères de fermentation dudit au moins un pentose, dans cette variante les présents inventeurs ont réalisé une étape d'élimination des levures « petites » car ce phénotype est incompatible avec les procédés de production de levures industrielles au sens de l'invention.

Les chercheurs ont constaté que l'étape d'évolution dirigée telle qu'expliquée précédemment a permis d'augmenter de manière non négligeable l'activité de la xylose isomérase qui s'est traduit par une augmentation de la vitesse de consommation du xylose.

Sans vouloir être lié par une théorie, cet effet inattendu semble dû à l'augmentation du nombre de copies XI dans la souche modifiée.

La présente invention a encore pour objet la souche de levure industrielle *Saccharomyces cerevisiae* EG6 directement obtenue par le procédé selon l'invention après l'étape d'évolution dirigée et qui consiste en la souche de levure déposée le 23 novembre 2010 à la C.N.C.M (Collection Nationale de Cultures de Microorganismes de l'Institut Pasteur, 25 rue du Docteur Roux, 75724 Paris, France) sous le N°I-4399 dans les conditions du traité de Budapest.

La présente invention a également pour objet la souche de levure industrielle *Saccharomyces cerevisiae* EG7 directement obtenue par le procédé selon l'invention après l'étape d'évolution dirigée déposée le 23 novembre 2010 à la C.N.C.M (Collection Nationale de Cultures de Microorganismes de l'Institut Pasteur) sous le N°I-4400 dans les conditions du traité de Budapest.

La présente invention a également pour objet la souche de levure industrielle *Saccharomyces cerevisiae* EG8 directement obtenue par le procédé selon l'invention après l'étape d'évolution dirigée déposée le 14 décembre 2010 à la C.N.C.M (Collection Nationale de Cultures de Microorganismes de l'Institut Pasteur) sous le N°I-4417 dans les conditions du traité de Budapest.

La présente invention a également pour objet la souche de levure industrielle *Saccharomyces cerevisiae* EG10 directement obtenue par le procédé selon l'invention après l'étape d'évolution dirigée déposée le 5 octobre 2011 à la C.N.C.M (Collection Nationale de Cultures de Microorganismes de l'Institut Pasteur) sous le N°I-4538 dans les conditions du traité de Budapest.

D'autres souches selon l'invention sont des souches dérivées à partir d'une ou plusieurs souches selon l'invention, par exemple à partir d'une souche ou plusieurs souches obtenues selon le procédé ci-dessus, et notamment à partir d'une ou plusieurs des souches déposées à la CNCM sous le numéro I-4399 le 23 novembre 2010, sous le numéro I-4400 le 23 novembre 2010, sous le numéro I-4417 le 14 décembre 2010 et sous le numéro I-4538 le 5 octobre 2011.

Par l'expression « *souche dérivée* », on désigne en particulier les souches dérivées par un ou plusieurs croisements et/ou par mutation et/ou par transformation génétique.

Les souches dérivées par croisement peuvent être obtenues par croisement d'une souche selon l'invention avec la même souche, ou une autre souche selon l'invention, ou une autre souche quelconque.

Les souches dérivées par mutation peuvent être des souches ayant subi au moins une mutation spontanée dans leur génome ou au moins une mutation induite par mutagenèse. La ou les mutations des souches dérivées peuvent être silencieuses ou non.

Par l'expression « *mutagenèse* », on désigne à la fois la mutagenèse aléatoire obtenue par l'application d'un rayonnement (par exemple UV), ou par des agents chimiques mutagènes, et la mutagenèse insertionnelle ou dirigée, par transposition ou par intégration d'un fragment d'ADN exogène.

Les souches dérivées entrant dans le cadre de l'exposé sont celles comportant au moins un gène XI exogène et un gène XDH exogène, et qui sont susceptibles de fermenter le xylose pour produire de l'éthanol, et notamment avec un rendement moyen d'éthanol produit sur xylose consommé supérieur ou égal à 0,2 g, de préférence 0,3 g, 0,35 g voire 0,38 g d'éthanol par g de xylose consommé.

Ces souches dérivées présentent en outre de préférence une délétion du gène *GRE3,* et / ou un contrôle des gènes *XKS1* et/ou *RPE1* et/ou *RKI1* et/ou *TKL1* et/ou *TAL1* sous le contrôle d'un promoteur d'un gène non réprimé par l'anaérobiose ou par la répression catabolique induite par une quelconque source de carbone, et fortement exprimé lors de la fermentation alcoolique, tel qu'un promoteur d'un gène codant une enzyme de la glycolyse ou codant une enzyme alcool déshydrogénase, de préférence le promoteur de *ADH1, PGK1, TDH3, PDC2* ou *GAL1*/*10.*

De préférence encore :
- la souche de levure *Saccharomyces cerevisiae* obtenue est pratiquement ou totalement exempte de marqueurs notamment de résistance à des antibiotiques.

De préférence, les souches de levure *Saccharomyces cerevisiae* préparées conformément à la présente invention, selon les critères ci-dessus définis, conservent, après introduction des modifications génétiques et autres mutations générées lors de l'étape d'évolution dirigée, leurs caractéristiques génotypiques et phénotypiques après un process industriel complet de production. En particulier, les levures produites présentent une cinétique de production d'alcool, une cinétique de consommation de xylose et/ou d'arabinose et une quantité maximale d'alcool produite rigoureusement identiques à la souche de levure avant l'application d'un process industriel complet.

Par ailleurs, les caractéristiques industrielles de la souche choisie avant manipulation telles que décrites précédemment (taux de croissance, rendement de production, aptitude au séchage) demeurent inchangées.

Le présent exposé a encore pour objet un procédé de production d'éthanol, à partir d'un milieu comportant au moins un pentose, par fermentation à l'aide d'une levure selon l'invention, mentionnée ci-dessus, ou telle qu'obtenue par un procédé conforme à l'invention comme il vient d'être décrit.

De manière préférée, le procédé de production d'éthanol présente les caractéristiques alternatives et/ou complémentaires suivantes :
- Ledit au moins un pentose est le xylose ou un mélange de xylose et d'arabinose.
- Ledit milieu est choisi dans le groupe constitué par, les hydrolysats de lignine, de cellulose, d'hémicellulose, de dextrines ou d'amidon.
- Dans le cas d'une SSF les vitesses moyennes de libération des hexoses, majoritairement du glucose, sont de l'ordre de 2,8 à 5,6g/L/h avec une concentration extracellulaire en hexose, majoritairement en glucose, nulle.
- Le rendement moyen d'éthanol produit sur xylose consommé est supérieur ou égal à 0,38 g d'éthanol par g de xylose consommé, par exemple il peut être approximativement de 0,40 g d'éthanol par g de xylose consommé.

Les concentrations en sucres pouvant être mises en oeuvre (par exemple 70g/kg de Xylose ou 150g/kg de Xylose) sont à la connaissance de la Demanderesse, les concentrations maximum que l'on peut rencontrer en pratique. Tous les essais publiés faisant référence à la fermentation du xylose ont été réalisés avec des concentrations nettement inférieures en sucres totaux.

D'autres caractéristiques et avantages de l'invention apparaîtront encore mieux à la lecture des exemples de réalisation qui suivent qui sont donnés à titre purement illustratif et non limitatif, et pour la compréhension desquels on se reportera aux dessins annexés.

### EXEMPLES

### Exemple 1

La sélection de la souche est telle que décrite dans la description ci-dessus.

Toutes les séquences d'ADN qui ont été utilisées pour les différentes transformations visant la surexpression d'un gène ont été obtenues à partir d'un vecteur-type connu (type pUC) dans lequel ont été déclinés :
- les cibles d'intégration ;
- les promoteurs/terminateurs choisis par gène d'intérêt et
- les marqueurs de résistance qui seront éliminés par la suite (voir ci-dessous).

Un exemple de vecteur utilisé pour la surexpression de la XDH de *Pichia stipitis* est illustré à la Figure 1.

Un exemple de vecteur utilisé pour la surexpression de la XI de *Clostridium phytofermentans* est illustré à la Figure 2.

Pour la disruption des copies du gène *GRE3* de la souche industrielle sélectionnée, les inventeurs ont utilisé des amplifiats PCR à partir d'un plasmide de type pUG6 (Güldener U, Heck S, Fielder T, Beinhauer J, Hegemann JH. Nucleic Acids Res. 1996 Jul 1;24(13):2519-24).

L'étape de transformation de la levure a été mise en oeuvre d'après Gietz, R.D. and R.A. Woods. (2002) TRANSFORMATION OF YEAST BY THE Liac/SS CARRIER DNA/PEG METHOD. Methods in Enzymology 350: 87-96.

Les souches de levure selon l'invention, respectivement EG6, EG7, EG8 et EG10 ont été déposées à la CNCM et il leur a été attribué les numéros°I-4399, I-4400, I-4417 et I-4538, respectivement.

Les souches conformes à l'invention :
- possèdent le génotype suivant :
   *Ethanol Red™*, *Delta GRE3, BUD5::pADH1-XKS1-tCYC1, TAL1::pPGK1-TAL1-tCYC1, TKL1::pTDH3-TKL1-tCYC1,RPE1::pTDH3-RPE1-tCYC1, RKI1::pTDH3-RKI1-tCYC1, HO::PsXYL2-HYGRO, BUD5::CpXI-BLAST*
- sont dépourvues de tout marqueur résiduel (par action de la cre recombinase).

### Exemple 2

La mutagenèse des souches obtenues à l'Exemple précédent a été réalisée de manière modérée à savoir de 100 à 500J/cm² et de préférence à 300J/cm² d'Ultra-violets à 254nm.

Après une semaine de culture à 32°C dans un milieu type YE (Yeast Extract 0,5%) contenant 7% de Xylose sous agitation, sans aération - la limitation en O₂ étant réalisée grâce à une surpression partielle dans les fioles due au CO₂ produit lors de la fermentation - un ml de la culture est utilisé pour ré-ensemencer le même milieu. Cette opération est répétée 6 fois. Les cellules sont finalement étalées sur milieu gélosé YE Glucose à 20g/L. Des colonies isolées sont prélevées puis cultivées successivement sur :
- YE Glycérol à 20g/L et en aérobiose pour éliminer les mutants « petite » i.e. déficients respiratoires ;
- YE Glucose pour vérifier leur vitesse de croissance ;
- YE Xylose pour identifier les clones les plus intéressants.

### Exemple 3

Après obtention de la souche EG6, la copie du gène XDH qui a été ajoutée à l'exemple 1 a été substituée par le gène de résistance à la kanamycine. La nouvelle souche obtenue est nommée EG6 - XDH. La capacité de la souche en question à fermenter le xylose a été comparée à celle de la souche EG6. Le résultat de cette comparaison est présenté sur la figure 4.

## Revendications

1. Souche de levure **caractérisée en ce qu'**elle comporte des gènes exogènes de la voie métabolique du xylose, dans laquelle les gènes exogènes de la voie métabolique du xylose présents dans la souche de levure consistent en au moins une copie d'un gène exogène codant pour une xylose isomérase, et une copie d'un gène exogène codant pour une xylitol déshydrogénase, et **en ce qu'**elle a subi une étape ultérieure d'évolution dirigée comportant les étapes successives suivantes consistant à soumettre ladite souche de levure à :
(i) une mutagenèse,
(ii) une croissance en cultures cycliques sous O₂ limité dans un milieu comportant au moins un pentose, notamment du xylose, et
(iii) une sélection par croissance aérobie sur milieu solide contenant comme seule source de carbone du glycérol.

2. Souche de levure selon la revendication 1, dans laquelle le gène exogène codant pour une xylose isomérase est un gène issu de *Clostridium, Piromyces, Bacteroides, Streptomyces, Haemophilus, Burkholdeha, Enterococcus, Thermotoga, Fusobactehum, Geobacillus, Arthrobacter, Ciona*, *Physcomitrella, Cellvibrio, Chitinophaga*, *Saccharopolyspora* ou *Salinibacter,* et de préférence est un gène issu de *Clostridium phytofermentans* ou de *Piromyces* sp. E2.

3. Souche de levure selon la revendication 1 ou la revendication 2, dans laquelle le gène exogène codant pour une xylitol déshydrogénase est issu de *Pichia stipitis.*

4. Souche de levure selon l'une quelconque des revendications 1 à 3, choisie parmi *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp. et *Debaryomyces* spp., et de préférence est une souche de *Saccharomyces cerevisiae.*

5. Souche de levure selon l'une quelconque des revendications 1 à 4, dans laquelle au moins une copie, de préférence au moins deux copies, d'un gène codant pour une aldose réductase est délété.

6. Souche de levure selon la revendication 5, dans laquelle le gène délété est *GRE3.*

7. Souche de levure selon l'une quelconque des revendications 1 à 6, dans laquelle un gène endogène codant pour une xylulokinase, de préférence le gène *XKS1,* est placé sous le contrôle d'un promoteur d'un gène non réprimé par l'anaérobiose ou par la répression catabolique induite par une quelconque source de carbone, et fortement exprimé lors de la fermentation alcoolique.

8. Souche de levure selon l'une quelconque des revendications 1 à 7, dans laquelle au moins un gène endogène de la partie non oxydative de la voie des pentoses phosphates, de préférence choisi parmi les gènes *RPE1*, *RKI1, TKL1* et *TAL 1,* et de manière particulièrement préférée la totalité de ces gènes, est placé sous le contrôle d'un promoteur d'un gène non réprimé par l'anaérobiose ou par la répression catabolique induite par une quelconque source de carbone, et fortement exprimé lors de la fermentation alcoolique.

9. Souche de levure selon l'une quelconque des revendications 1 à 8, dans laquelle le promoteur est le promoteur d'un gène codant pour une enzyme de la glycolyse ou codant pour une enzyme alcool déshydrogénase, de préférence le promoteur de *ADH1, PGK1, TDH3, PDC2* ou *GAL1*/*10.*

10. Souche de levure selon l'une quelconque des revendications 1 à 9, **caractérisée en ce qu'**elle est une souche aneuploïde ou polyploïde et / ou une souche prototrophe.

11. Souche de levure selon l'une quelconque des revendications 1 à 10, **caractérisée en ce qu'**elle comprend au moins deux copies du gène exogène codant pour une xylose isomérase, de préférence au moins trois copies ou au moins quatre copies du gène exogène codant pour une xylose isomérase.

12. Souche de levure selon l'une quelconque des revendications 1 à 11, **caractérisée en ce qu'**elle est une souche industrielle présentant une résistance aux inhibiteurs de fermentation issus de l'hydrolyse de la biomasse tels que les produits phénoliques, le furfural ou l'acide acétique.

13. Souche de levure selon l'une quelconque des revendications 1 à 12, **caractérisée en ce qu'**elle produit une concentration d'éthanol d'au moins 16 %, de préférence d'au moins 17% v/v, sur un hydrolysat de céréales, en conditions de saccharification et fermentation simultanée à 32 °C.

14. Souche de levure selon l'une quelconque des revendications 1 à 13, **caractérisée en ce qu'**elle est choisie dans le groupe consistant en la souche de *Saccharomyces cerevisiae* déposée à la CNCM le 23 novembre 2010, sous le N ° l-4399, la souche de *Saccharomyces cerevisiae* déposée à la CNCM le 23 novembre 2010, sous le N °l-4400, la souche de *Saccharomyces cerevisiae* déposée à la CNCM le 14 décembre 2010, sous le N ° 1-4417, et la souche de *Saccharomyces cerevisiae* déposée à la CNCM le 5 octobre 2011, sous le N ° l-4538.

15. Souche de levure selon l'une quelconque des revendications 1 à 13, **caractérisée en ce qu'**elle est une souche dérivée d'une ou plusieurs des souches définies dans la revendication 14.

16. Procédé de préparation d'une souche de levure, comprenant l'introduction dans une souche de levure de départ de gènes exogènes de la voie métabolique du xylose, dans lequel les gènes exogènes de la voie métabolique du xylose introduits dans la souche de levure de départ consistent en au moins une copie d'un gène exogène codant pour une xylose isomérase, et au moins une copie d'un gène exogène codant pour une xylitol déshydrogénase, et comprenant en outre une étape ultérieure d'évolution dirigée comportant les étapes successives suivantes consistant à soumettre la levure obtenue à :
(i) une mutagenèse,
(ii) une croissance en cultures cycliques sous O₂ limité dans un milieu comportant au moins un pentose, notamment du xylose, et
(iii) une sélection par croissance aérobie sur milieu solide contenant comme seule source de carbone du glycérol,
de sorte à procurer des mutants non-déficients respiratoires de ladite levure qui présentent une croissance en anaérobiose en présence d'un milieu comportant ledit au moins un pentose.

17. Procédé selon la revendication 16, comprenant en outre la délétion d'au moins une copie, de préférence au moins deux copies, d'un gène codant pour une aldose réductase, de préférence le gène *GRE3,* dans la souche de départ.

18. Procédé selon la revendication 16 ou la revendication 17, de préparation d'une souche de levure selon l'une quelconque des revendications 1 à 15.

19. Procédé selon l'une quelconque des revendications 16 à 18, de préparation d'une souche de levure *Saccharomyces cerevisiae* apte à produire de l'éthanol à partir d'un milieu comportant du xylose et qui comprend les étapes suivantes consistant à :
(i) sélectionner une souche de *Saccharomyces cerevisiae,*
(ii) intégrer les cassettes d'expression suivantes dans le génome de la levure de l'étape (i),
a. l'association du type cadre ouvert de lecture (ORF) d'un gène codant pour une xylose isomérase sous la dépendance d'un promoteur et d'un terminateur de *Saccharomyces cerevisiae,* ladite cassette étant flanquée en amont et en aval de régions recombinogéniques permettant son intégration ciblée dans le génome,
b. l'association du type cadre ouvert de lecture (ORF) d'un gène codant pour une xylitol déshydrogénase sous la dépendance d'un promoteur et d'un terminateur de *Saccharomyces cerevisiae,* ladite cassette étant flanquée en amont et en aval de régions recombinogéniques permettant son intégration ciblée dans le génome,
(iii) induire l'expression d'au moins un gène de chaque étape de la partie non oxydative de la voie des pentoses phosphate ainsi qu'au moins un gène codant pour la xylulokinase (XKS1) en les plaçant sous la dépendance d'un promoteur d'un gène non réprimé ni par l'anaérobiose ni par la répression catabolique induite par une quelconque source de carbone, et fortement exprimé lors de la fermentation alcoolique, et
(iv) déléter au moins deux copies du cadre ouvert de lecture (ORF) du gène de *Saccharomyces cerevisiae GRE3* codant pour une aldose réductase.

20. Procédé selon la revendication 19, **caractérisé en ce que** le gène codant pour une xylose isomérase est un gène XI codant pour l'enzyme Xylose Isomérase choisi parmi ceux présents dans les génomes de *Clostridium, Piromyces*, *Bacteroides, Streptomyces, Haemophilus, Burkholderia, Enterococcus, Thermotoga*, *Fusobacterium, Geobacillus, Arthrobacter, Ciona*, *Physcomitrella*, *Cellvibrio, Chitinophaga*, *Saccharopolyspora, Salinibacter.*

21. Procédé selon la revendication 19 ou la revendication 20, **caractérisé en ce que** ledit gène XI est choisi parmi un gène de *Clostridium phytofermentans* ou de *Piromyces sp.* E2.

22. Procédé selon la revendication 19 ou la revendication 20, **caractérisé en ce que** le gène codant pour une xylitol deshydrogénase est un gène de *Pichia stipitis* codant pour l'enzyme xylitol déshydrogénase.

23. Procédé selon l'une quelconque des revendications 19 à 21, **caractérisé en ce que** la souche de levure de l'étape (i) présente une activité xylitol déshydrogénase XDH endogène inférieure à 150mKat/g de protéines.

24. Procédé selon l'une quelconque des revendications 19 à 23, **caractérisé en ce que** la souche de levure de l'étape (i) est sélectionnée parmi les souches industrielles présentant une résistance aux inhibiteurs de fermentation issus de l'hydrolyse de la biomasse tels que les produits phénoliques, le furfural ou l'acide acétique.

25. Procédé selon l'une quelconque des revendications 19 à 24, **caractérisé en ce que** le promoteur de *Saccharomyces cerevisiae* de l'étape (iii) est choisi dans le groupe comprenant les promoteurs des gènes codant pour les enzymes de la glycolyse et ceux codant pour les enzymes alcool déshydrogénase.

26. Procédé selon l'une quelconque des revendications 19 à 25, **caractérisé en ce que** ledit groupe est constitué par *ADH1*, *PGK1*, *TDH3, PDC2* et GAL1/10, de préférence *ADH1*, et **en ce que** le terminateur de *Saccharomyces cerevisiae* est constitué par *CYC1* ou par le terminateur propre du gène de la voie non oxydative des pentoses phosphates.

27. Procédé selon l'une quelconque des revendications 19 à 26, **caractérisé en ce que** la souche de l'étape (i) est une souche industrielle choisie parmi les souches aptes à produire de fortes concentrations d'éthanol, d'au moins 17% v/v, sur un hydrolysat de céréales, en conditions de Saccharification et fermentation Simultanée (SSF) et à 32 °C.

28. Procédé selon l'une quelconque des revendications 19 à 27, **caractérisé en ce qu'**il comprend en outre une ou plusieurs étapes d'insertion de marqueurs de résistance aux antibiotiques, une ou plusieurs étapes de sélection de souches selon le critère de leur résistance aux antibiotiques, et une ou plusieurs étapes d'excision des marqueurs de résistance aux antibiotiques.

29. Procédé de production d'éthanol, à partir d'un milieu comprenant du xylose, par fermentation d'une levure selon l'une quelconque des revendications 1 à 15 ou d'une levure obtenue par un procédé selon l'une quelconque des revendications 16 à 28.

30. Procédé selon la revendication 29 **caractérisé en ce que** le milieu comprend un mélange de xylose et d'arabinose.

31. Procédé selon la revendication 29 ou la revendication 30, **caractérisé en ce que** ledit milieu est choisi dans le groupe constitué par les hydrolysats de lignine, de cellulose, d'hémicellulose, de l'amidon.

## Patentansprüche

1. Hefestamm, **dadurch gekennzeichnet, dass** er exogene Gene des Xylose-Stoffwechselwegs umfasst, wobei die exogenen Gene exogene Gene des Xylose-Stoffwechselwegs in dem Hefestamm aus wenigstens einer Kopie eines exogenen Gens bestehen, das für eine Xylose-Isomerase kodiert, sowie eine Kopie eines exogenen Gens, das für eine Xylitol-Dehydrogenase kodiert, und dadurch, dass er einen gesteuerten letzten Entwicklungsschritt durchlaufen hat, welcher die folgenden aufeinanderfolgenden Schritte umfasst, bei welchen der Hefestamm folgendem unterzogen wird:
(i) einer Mutagenese,
(ii) einem Wachstum in zyklischen Kulturen unter begrenztem O₂ in einem Milieu, welches wenigstens eine Pentose umfasst, insbesondere Xylose, und
(iii) einer Selektion durch aerobes Wachstum auf festem Milieu, umfassend Glycerin als einzige Kohlenstoffquelle.

2. Hefestamm nach Anspruch 1, bei welchem das für eine Xylose-Isomerase kodierende exogene Gen ein aus *Clostridium, Piromyces, Bacteroides, Streptomyces, Haemophilus, Burkholdeha, Enterococcus, Thermotoga, Fusobactehum, Geobacillus, Arthrobacter, Ciona, Physcomitrella, Cellvibrio, Chitinophaga, Saccharopolyspora* oder *Salinibacter* stammendes Gen ist und vorzugsweise ein aus *Clostridium phytofermentans* oder *Piromyces* sp. E2 stammendes Gen ist.

3. Hefestamm nach Anspruch 1 oder Anspruch 2, wobei das für eine Xylitol-Dehydrogenase kodierende exogene Gen ein aus *Pichia stipitis* stammendes Gen ist.

4. Hefestamm nach einem der Ansprüche 1 bis 3, gewählt aus *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp. und *Debaryomyces* spp., und welcher vorzugsweise ein Stamm aus *Saccharomyces cerevisiae* ist.

5. Hefestamm nach einem der Ansprüche 1 bis 4, bei welchem wenigstens eine Kopie, vorzugsweise wenigstens zwei Kopien eines für eine Aldose-Reduktase kodierenden Gens deletiert ist/sind.

6. Hefestamm nach Anspruch 5, bei welchem das deletierte Gen *GRE3* ist.

7. Hefestamm nach einem der Ansprüche 1 bis 6, bei welchem ein für eine Xylulokinase kodierendes endogenes Gen, vorzugsweise das Gen *XKS1,* der Regulierung durch einen Promotor eines Gens unterliegt, das nicht durch Anaerobiose oder durch katabolische Repression durch eine Kohlenstoffquelle reprimiert und während der alkoholischen Gärung stark exprimiert wird.

8. Hefestamm nach einem der Ansprüche 1 bis 7, bei welchem wenigstens ein endogenes Gen des nicht-oxidativen Teils des Pentosephosphatwegs, vorzugsweise gewählt aus den Genen *RPE1, RKI1, TKL1* und *TAL 1,* und besonders bevorzugt die Gesamtheit dieser Gene, der Regulierung durch einen Promotor eines Gens unterliegt, das nicht durch Anaerobiose oder durch katabolische Repression durch eine Kohlenstoffquelle reprimiert und während der alkoholischen Gärung stark exprimiert wird.

9. Hefestamm nach einem der Ansprüche 1 bis 8, bei welchem der Promotor der Promotor eines Gens ist, das für ein Enzym der Glykolyse kodiert oder das für ein Enzym der Alkohol-Dehydrogenase kodiert, vorzugsweise der Promotor von *ADH1, PGK1, TDH3, PDC2* oder *GAL1*/*10.*

10. Hefestamm nach einem der Ansprüche 1 bis 9, **dadurch gekennzeichnet, dass** es sich um einen aneuploiden oder polyploiden Stamm und/oder einen porotrophen Stamm handelt.

11. Hefestamm nach einem der Ansprüche 1 bis 10, **dadurch gekennzeichnet, dass** er wenigstens zwei Kopien des exogenen Gens umfasst, welches für eine Xylose-Isomerase kodiert, vorzugsweise wenigstens drei Kopien oder wenigstens vier Kopien des exogenen Gens umfasst, welches für eine Xylose-Isomerase kodiert.

12. Hefestamm nach einem der Ansprüche 1 bis 11, **dadurch gekennzeichnet, dass** es sich um einen industriellen Stamm handelt, der eine Resistenz gegen Gärungsinhibitoren aufweist, die aus der Hydrolyse von Biomasse stammen, wie beispielsweise Phenolharzprodukte, Furfural oder Essigsäure.

13. Hefestamm nach einem der Ansprüche 1 bis 12, **dadurch gekennzeichnet, dass** er eine Ethanolkonzentration von wenigstens 16%, vorzugsweise wenigstens 17% v/v, auf einem Getreidehydrolysat bei gleichzeitiger Verzuckerung und Gärung bei 32 °C erzeugt.

14. Hefestamm nach einem der Ansprüche 1 bis 13, **dadurch gekennzeichnet, dass** er gewählt ist aus der Gruppe, umfassend den *Saccharomyces cerevisiae-Stamm,* der bei der CNCM am 23. November 2010 unter der Nr. 1-4399 hinterlegt wurde, den *Saccharomyces cerevisiae-Stamm,* der bei der CNCM am 23. November 2010 unter der Nr. 1-4400 hinterlegt wurde, den *Saccharomyces cerevisiae-Stamm,* der bei der CNCM am 14. Dezember 2010 unter der Nr. 1-4417 hinterlegt wurde, und den *Saccharomyces cerevisiae-*Stamm, der bei der CNCM am 5. Oktober 2011 unter der Nr. 1-4538 hinterlegt wurde.

15. Hefestamm nach einem der Ansprüche 1 bis 13, **dadurch gekennzeichnet, dass** es sich um einen Stamm handelt, der von einem oder mehreren der in Anspruch 14 definierten Stämme abgeleitet wurde.

16. Herstellungsverfahren für einen Hefestamm, welches die Einführung exogener Gene des Xylose-Stoffwechselwegs in einen Ausgangs-Hefestamm umfasst, wobei die exogenen Gene des Xylose-Stoffwechselwegs, die in den Ausgangs-Hefestamm eingeführt werden, aus wenigstens einer Kopie eines exogenen Gens bestehen, das für eine Xylose-Isomerase kodiert, und wenigstens eine Kopie eines exogenen Gens, das für eine Xylitol-Dehydrogenase kodiert, und welches ferner einen gesteuerten letzten Entwicklungsschritt umfasst, welcher die folgenden aufeinanderfolgenden Schritte umfasst, bei welchen die erhaltene Hefe folgendem unterzogen wird:
(i) einer Mutagenese,
(ii) einem Wachstum in zyklischen Kulturen unter begrenztem O₂ in einem Milieu, welches wenigstens eine Pentose umfasst, insbesondere Xylose, und
(iii) einer Selektion durch aerobes Wachstum auf festem Milieu, umfassend Glycerin als einzige Kohlenstoffquelle,
zur Lieferung nicht-defizienter respiratorischer Mutanten der Hefe, die ein aerobes Wachstum in Anwesenheit eines Milieus aufweisen, welches die wenigstens eine Pentose umfasst.

17. Verfahren nach Anspruch 16, welches ferner die Deletion wenigstens einer Kopie, vorzugsweise von wenigstens zwei Kopien, eines Gens umfasst, welches in dem Ausgangsstamm für eine Aldose-Reduktase kodiert, vorzugsweise des Gens *GRE3.*

18. Verfahren nach Anspruch 16 oder Anspruch 17 zur Herstellung eines Hefestamms nach einem der Ansprüche 1 bis 15.

19. Verfahren nach einem der Ansprüche 16 bis 18 zur Herstellung eines *Saccharomyces* cerevisiae-Hefestamms, der dazu geeignet ist, Ethanol auf der Basis eines Xylose-haltigen Milieus zu erzeugen, und welches die folgenden Schritte umfasst:
(i) Selektieren eines *Saccharomyces cerevisiae*-Stamms,
(ii) Integrieren folgender Expressionskassetten in das Genom der Hefe aus Schritt (i),
a. Verbindung vom Typ Offener Leserahmen (ORF) eines für eine Xylose-Isomerase kodierenden Gens in Abhängigkeit von einem Promotor und einem Terminator von *Saccharomyces cerevisiae,* wobei die Kassette stromaufwärts und stromabwärts von rekombinogenen Bereichen umgeben ist, die ihre gezielte Integration in das Genom ermöglichen,
b. Verbindung vom Typ Offener Leserahmen (ORF) eines für eine Xylitol-Dehydrogenase kodierenden Gens in Abhängigkeit von einem Promotor und einem Terminator von *Saccharomyces cerevisiae,* wobei die Kassette stromaufwärts und stromabwärts von rekombinogenen Bereichen umgeben ist, die ihre gezielte Integration in das Genom ermöglichen,
(iii) Induzieren der Expression wenigstens eines Gens jedes Schrittes des nicht-oxidativen Teils des Pentosephosphatwegs sowie wenigstens eines für die Xylulokinase (XKS1) kodierenden Gens, indem diese in die Abhängigkeit von einem Promotor eines Gens gebracht werden, das nicht reprimiert wird, weder durch Anaerobiose noch durch katabolische Repression durch eine beliebige Kohlenstoffquelle, und bei der alkoholischen Gärung stark exmprimiert wird, und
(iv) Deletieren von wenigstens zwei Kopien des Offenen Leserahmens (ORF) des *Saccharomyces cerevisiae-GRE3-Gens,* welches für eine Aldose-Reduktase kodiert.

20. Verfahren nach Anspruch 19, **dadurch gekennzeichnet, dass** das für eine Xylose-Isomerase kodierende Gen ein Gen XI ist, welches für das Enzym Xylose-Isomerase kodiert, gewählt aus Genen, welche in den Genomen von *Clostridium, Piromyces, Bacteroides, Streptomyces, Haemophilus, Burkholderia, Enterococcus, Thermotoga, Fusobacterium, Geobacillus, Arthrobacter, Ciona, Physcomitrella, Cellvibrio, Chitinophaga, Saccharopolyspora, Salinibacter vorl iegen.*

21. Verfahren nach Anspruch 19 oder Anspruch 20, **dadurch gekennzeichnet, dass** das Gen XI gewählt ist aus einem Gen von *Clostridium phytofermentans* oder *Piromyces sp.* E2.

22. Verfahren nach Anspruch 19 oder Anspruch 20, **dadurch gekennzeichnet, dass** das für eine Xylitol-Dehydrogenase kodierende Gen ein Gen von *Pichia stipitis* ist, welches für das Enzym Xylitol-Dehydrogenase kodiert.

23. Verfahren nach einem der Ansprüche 19 bis 21, **dadurch gekennzeichnet, dass** der Hefestamm aus Schritt (i) eine endogene Xylitol-Dehydrogenase-Aktivität XDH unter 150mKat/g Proteine aufweist.

24. Verfahren nach einem der Ansprüche 19 bis 23, **dadurch gekennzeichnet, dass** der Hefestamm aus Schritt (i) aus industriellen Stämmen gewählt wird, welche eine Resistenz gegen Gärungsinhibitoren aufweisen, die aus der Hydrolyse von Biomasse stammen, wie beispielsweise Phenolharzprodukte, Furfural oder Essigsäure.

25. Verfahren nach einem der Ansprüche 19 bis 24, **dadurch gekennzeichnet, dass** der Promotor von *Saccharomyces cerevisiae* aus Schritt (iii) aus der Gruppe gewählt wird, die Promotoren der Gene umfasst, die für Glycolyse-Enzyme kodieren und jene, die für Akohol-Dehydrogenase-Enzyme kodieren.

26. Verfahren nach einem der Ansprüche 19 bis 25, **dadurch gekennzeichnet, dass** die Gruppe *ADH1, PGK1, TDH3, PDC2* und GAL1/10 umfasst, vorzugsweise *ADH1,* und dadurch, dass der Terminator von *Saccharomyces cerevisiae CYC1* umfasst oder den Terminator des Gens des nicht-oxidativen Teils des Pentosephosphatwegs.

27. Verfahren nach einem der Ansprüche 19 bis 26, **dadurch gekennzeichnet, dass** der Hefestamm aus Schritt (i) ein industrieller Stamm ist, der aus denjenigen Stämmen gewählt wird, die dazu geeignet sind, hohe Ethanolkonzentrationen von wenigstens 17% v/v auf einem Getreidehydrolysat bei gleichzeitiger Verzuckerung und Gärung (SSF) bei 32 °C zu erzeugen.

28. Verfahren nach einem der Ansprüche 19 bis 27, **dadurch gekennzeichnet, dass** es ferner einen oder mehrere Schritte der Einführung von Markern für Antibiotikaresistenzen, einen oder mehrere Schritte der Selektion von Stämmen nach dem Kriterium ihrer Antibiotikaresistenz sowie einen oder mehrere Schritte der Exzision von Antibiotikaresistenz-Markern umfasst.

29. Verfahren zur Herstellung von Ethanol auf der Basis eines Xylose-haltigen Milieus durch Gärung einer Hefe nach einem der Ansprüche 1 bis 15 oder einer Hefe, die durch ein Verfahren nach einem der Ansprüche 16 bis 28 gewonnen wird.

30. Verfahren nach Anspruch 29, **dadurch gekennzeichnet, dass** das Milieu eine Mischung aus Xylose und Arabinose umfasst.

31. Verfahren nach Anspruch 29 oder Anspruch 30, **dadurch gekennzeichnet, dass** das Milieu gewählt wird aus der Gruppe, umfassend Lignin-, Zellulose-, Hemizellulose und Stärke-Hydrolysate.

## Claims

1. Yeast strain, **characterized in that** it comprises exogenous genes of the xylose metabolic pathway, wherein the exogenous genes of the xylose metabolic pathway present in the yeast strain consist of at least one copy of an exogenous gene encoding a xylose isomerase, and a copy of an exogenous gene encoding a xylitol dehydrogenase, and **in that** it has undergone a subsequent step of directed evolution comprising the following successive steps consisting in subjecting said yeast strain to:
(i) a mutagenesis,
(ii) a growth in cyclic cultures, under limited O₂ in a medium comprising at least one pentose, especially xylose, and
(iii) a selection by aerobic growth on solid medium containing glycerol as sole carbon source.

2. Yeast strain according to Claim 1, wherein the exogenous gene encoding a xylose isomerase is a gene derived from *Clostridium, Piromyces, Bacteroides, Streptomyces, Haemophilus, Burkholderia, Enterococcus*, *Thermotoga, Fusobacterium, Geobacillus, Arthrobacter, Ciona, Physcomitrella*, *Cellvibrio, Chitinophaga*, *Saccharopolyspora* or *Salinibacter*, and is preferably a gene derived from *Clostridium phytofermentans* or *Piromyces* sp. E2.

3. Yeast strain according to Claim 1 or Claim 2, wherein the exogenous gene encoding a xylitol dehydrogenase is derived from *Pichia stipitis.*

4. Yeast strain according to any one of Claims 1 to 3, chosen from *Saccharomyces* spp., *Schizosaccharomyces* spp., *Pichia* spp., *Paffia* spp., *Kluyveromyces* spp., *Candida* spp., *Talaromyces* spp., *Brettanomyces* spp., *Pachysolen* spp. and *Debaromyces spp.,* and is preferably a strain of *Saccharomyces cerevisiae.*

5. Yeast strain according to any one of Claims 1 to 4, wherein at least one copy, preferably at least two copies, of a gene encoding aldose reductase is deleted.

6. Yeast strain according to Claim 5, wherein the deleted gene is *GRE3.*

7. Yeast strain according to any one of Claims 1 to 6, wherein an endogenous gene encoding xylulokinase, preferably the *XKS1* gene, is placed under the control of a promoter for a gene not repressed by anaerobic conditions or by catabolic repression induced by any carbon source, and strongly expressed during alcoholic fermentation.

8. Yeast strain according to any one of Claims 1 to 7, wherein at least one endogenous gene of the non-oxidative portion of the pentose phosphate pathway, preferably chosen from the *RPE1*, *RKI1, TKL1* and *TAL1* genes, and in particular preferably all of these genes, is placed under the control of a promoter for a gene not repressed by anaerobic conditions or by catabolic repression induced by any carbon source, and strongly expressed during alcoholic fermentation.

9. Yeast strain according to any one of Claims 1 to 8, wherein the promoter is the promoter for a gene encoding an enzyme for glycolysis or encoding an alcohol dehydrogenase enzyme, preferably the promoter for *ADH1*, *PGK1, TDH3, PDC2* or *GAL1*/*10.*

10. Yeast strain according to any one of Claims 1 to 9, **characterized in that** it is an aneuploid or polyploid strain and/or a phototrophic strain.

11. Yeast strain according to any one of Claims 1 to 10, **characterized in that** it comprises at least two copies of the exogenous gene encoding a xylose isomerase, preferably at least three copies or at least four copies of the exogenous gene encoding a xylose isomerase.

12. Yeast strain according to any one of Claims 1 to 11, **characterized in that** it is an industrial strain having a resistance to fermentation inhibitors derived from biomass hydrolysis, such as phenolic products, furfural or acetic acid.

13. Yeast strain according to any one of Claims 1 to 12, **characterized in that** it produces an ethanol concentration of at least 16%, preferably of at least 17% v/v, on a cereal hydrolysate, under simultaneous saccharification and fermentation conditions at 32°C.

14. Yeast strain according to any one of Claims 1 to 13, **characterized in that** it is chosen from the group consisting of the strain of *Saccharomyces cerevisiae* deposited at the CNCM [National Collection of Microorganism Cultures] on 23^{rd} November 2010 under number I-4399, the strain of *Saccharomyces cerevisiae* deposited at the CNCM on 23^{rd} November 2010 under number I-4400, the strain of *Saccharomyces cerevisiae* deposited at the CNCM on 14^{th} December 2010 under number I-4417, and the strain of *Saccharomyces cerevisiae* deposited at the CNCM on 5^{th} October 2011 under number I-4538.

15. Yeast strain according to any one of Claims 1 to 13, **characterized in that** it is a strain derived from one or more of the strains defined in Claim 14.

16. Method for preparing a yeast strain, comprising the introduction, into a starting yeast strain, of exogenous genes of the xylose metabolic pathway, wherein the exogenous genes of the xylose metabolic pathway introduced into said starting yeast strain consist of at least one copy of an exogenous gene encoding a xylose isomerase, and at least one copy of an exogenous gene encoding a xylitol dehydrogenase, and also comprising a subsequent step of directed evolution comprising the following successive steps consisting in subjecting the yeast obtained to:
(i) a mutagenesis,
(ii) a growth in cyclic cultures, under limited O₂ in a medium comprising at least one pentose, especially xylose, and
(iii) a selection by aerobic growth on solid medium containing glycerol as sole carbon source,
so as to obtain non-respiratory-deficient mutants of said yeast which grow under anaerobic conditions in the presence of a medium comprising said at least one pentose.

17. Method according to Claim 16, also comprising the deletion of at least one copy, preferably at least two copies, of a gene encoding aldose reductase, preferably the *GRE3* gene, in the starting strain.

18. Method according to Claim 16 or Claim 17 for preparing a yeast strain according to any one of Claims 1 to 15.

19. Method according to any one of Claims 16 to 18 for preparing a *Saccharomyces cerevisiae* yeast strain able to produce ethanol from a medium comprising xylose and which comprises the following steps consisting in:
(i) selecting a strain of *Saccharomyces cerevisiae,*
(ii) integrating the following expression cassettes into the genome of the yeast from step (i),
a. the open reading frame (ORF)-type combination of a gene encoding a xylose isomerase under the control of a *Saccharomyces cerevisiae* promoter and terminator, said cassette being flanked upstream and downstream by recombinogenic regions enabling the targeted integration thereof into the genome,
b. the open reading frame (ORF)-type combination of a gene encoding a xylitol dehydrogenase under the control of a *Saccharomyces cerevisiae* promoter and terminator, said cassette being flanked upstream and downstream by recombinogenic regions enabling the targeted integration thereof into the genome,
(iii) inducing the expression of at least one gene for each step of the non-oxidative portion of the pentose phosphate pathway, and also at least one gene encoding xylulokinase (XKS1) by placing them under the control of a promoter for a gene not repressed either by anaerobic conditions or by catabolic repression induced by any carbon source, and strongly expressed during alcoholic fermentation, and
(iv) deleting at least two copies of the open reading frame (ORF) of the *Saccharomyces cerevisiae GRE3* gene encoding an aldose reductase.

20. Method according to Claim 19, **characterized in that** the gene encoding a xylose isomerase is an XI gene encoding the enzyme Xylose Isomerase, chosen from those present in the genomes of *Clostridium, Piromyces, Bacteroides, Streptomyces, Haemophilus, Burkholderia, Enterococcus, Thermotoga, Fusobacterium, Geobacillus, Arthrobacter, Ciorta, Plyscomitrella, Cellvibrio, Chitirtophaga, Saccharopolyspora* or *Salinibacter.*

21. Method according to Claim 19 or Claim 20, **characterized in that** said XI gene is chosen from a gene of *Clostridium phytofermentans* or *Piromyces* sp. E2.

22. Method according to Claim 19 or Claim 20, **characterized in that** the gene encoding a xylitol dehydrogenase is a gene of *Pichia stipitis* encoding the enzyme xylitol dehydrogenase.

23. Method according to any one of Claims 19 to 21, **characterized in that** the yeast strain from step (i) has an endogenous xylitol dehydrogenase XDH activity of less than 150 mkat/g of proteins.

24. Method according to any one of Claims 19 to 23, **characterized in that** the yeast strain from step (i) is selected from industrial strains having a resistance to fermentation inhibitors derived from biomass hydrolysis, such as phenolic products, furfural or acetic acid.

25. Method according to any one of Claims 19 to 24, **characterized in that** the *Saccharomyces cerevisiae* promoter from step (iii) is chosen from the group comprising promoters for the genes encoding enzymes for glycolysis and those encoding alcohol dehydrogenase enzymes.

26. Method according to any one of Claims 19 to 25, **characterized in that** said group consists of *ADH1, PGK1, TDH3, PDC2* and *GAL1*/*10,* preferably *ADH1,* and **in that** the *Saccharomyces cerevisiae* terminator consists of *CYC1* or the terminator belonging to the gene of the non-oxidative pentose phosphate pathway.

27. Method according to any one of Claims 19 to 26, **characterized in that** the strain from step (i) is an industrial strain chosen from strains able to produce high ethanol concentrations of at least 17% v/v, on a cereal hydrolysate, under simultaneous saccharification and fermentation (SSF) conditions and at 32°C.

28. Method according to any one of Claims 19 to 27, **characterized in that** it also comprises one or more steps of insertion of antibiotic resistance markers, one or more steps of selection of strains according to the criteria of their antibiotic resistance, and one or more steps of the excision of the antibiotic resistance markers.

29. Method for producing ethanol from a medium comprising xylose, by fermenting a yeast according to any one of Claims 1 to 15 or a yeast obtained by a method according to any one of Claims 16 to 28.

30. Method according to Claim 29, **characterized in that** the medium comprises a mixture of xylose and arabinose.

31. Method according to Claim 29 or Claim 30, **characterized in that** said medium is chosen from the group consisting of hydrolysates of lignin, of cellulose, of hemicellulose and of starch.


### SEQUENCE LISTING

<110> Lesaffre et Compagnie
<120> A method for preparing an industrial yeast, industrial yeast and application to the production of ethanol from at least one pentose
<130> 3873-02101
<160> 2
<170> PatentIn version 3.5
<210> 1
   <211> 1317
   <212> DNA
   <213> Clostridium phytofermentans
<400> 1
   atgaaaaatt actttccaaa tgttccagaa gtaaaatacg aaggcccaaa ttcaacgaat      60
   ccatttgctt ttaaatatta tgacgcaaat aaagttgtag cgggtaaaac aatgaaagag     120
   cactgtcgtt ttgcattatc ttggtggcat actctttgtg caggtggtgc tgatccattc     180
   ggtgtaacaa ctatggatag aacctacgga aatatcacag atccaatgga acttgctaag     240
   gcaaaagttg acgctggttt cgaattaatg actaaattag gaattgaatt cttctgtttc     300
   catgacgcag atattgctcc agaaggtgat acttttgaag agtcaaagaa gaatcttttt     360
   gaaatcgttg attacatcaa agagaagatg gatcagactg gtatcaagtt attatggggt     420
   actgctaata actttagtca tccaagattt atgcatggtg cttccacatc ttgcaacgca     480
   gacgtatttg catatgctgc tgctaagatt aagaatgcat tagatgcaac aattaaatta     540
   ggcggtaaag gttatgtatt ctggggtggt cgtgaaggtt atgaaacact tcttaataca     600
   gatttaggac ttgagcttga taatatggct agacttatga agatggctgt agagtatggc     660
   cgtgcaaatg gttttgatgg cgacttctat attgagccaa agccaaagga accaaccaag     720
   catcaatatg attttgatac agcaaccgta cttgctttcc ttcgcaaata tggcttagaa     780
   aaagatttca agatgaacat tgaagcaaac catgctactc ttgcaggtca tacctttgaa     840
   catgaacttg caatggctag agttaatggt gcatttggtt ctgtagatgc aaaccagggt     900
   gatccaaacc ttggatggga tacggatcaa ttcccaactg atgttcatag tgcaactctt     960
   gcaatgcttg aagtacttaa ggctggtgga ttcactaacg gcggacttaa ctttgatgca    1020
   aaggtaagac gtggttcctt cgaatttgat gatattgcat acggttatat tgcaggaatg    1080
   gatacttttg cacttggttt aattaaggct gctgagatta tcgacgatgg tagaatcgca    1140
   aaatttgtag atgatcgtta tgcaagctat aaaacaggaa ttggtaaagc aattgtggat    1200
   ggaactacat ctcttgaaga attagagcag tatgttttaa cacatagtga accagtaatg    1260
   cagagtggtc gtcaggaagt tcttgaaaca atcgtaaata atattttatt tagataa       1317
<210> 2
   <211> 438
   <212> PRT
   <213> Clostridium phytofermentans
<400> 2
   Met Lys Asn Tyr Phe Pro Asn Val Pro Glu Val Lys Tyr Glu Gly Pro
   1               5                   10                  15
   Asn Ser Thr Asn Pro Phe Ala Phe Lys Tyr Tyr Asp Ala Asn Lys Val
               20                  25                  30
   Val Ala Gly Lys Thr Met Lys Glu His Cys Arg Phe Ala Leu Ser Trp
           35                  40                  45
   Trp His Thr Leu Cys Ala Gly Gly Ala Asp Pro Phe Gly Val Thr Thr
       50                  55                  60
   Met Asp Arg Thr Tyr Gly Asn Ile Thr Asp Pro Met Glu Leu Ala Lys
   65                  70                  75                  80
   Ala Lys Val Asp Ala Gly Phe Glu Leu Met Thr Lys Leu Gly Ile Glu
                   85                  90                  95
   Phe Phe Cys Phe His Asp Ala Asp Ile Ala Pro Glu Gly Asp Thr Phe
               100                 105                 110
   Glu Glu Ser Lys Lys Asn Leu Phe Glu Ile Val Asp Tyr Ile Lys Glu
           115                 120                 125
   Lys Met Asp Gln Thr Gly Ile Lys Leu Leu Trp Gly Thr Ala Asn Asn
       130                 135                 140
   Phe Ser His Pro Arg Phe Met His Gly Ala Ser Thr Ser Cys Asn Ala
   145                 150                 155                 160
   Asp Val Phe Ala Tyr Ala Ala Ala Lys Ile Lys Asn Ala Leu Asp Ala
                   165                 170                 175
   Thr Ile Lys Leu Gly Gly Lys Gly Tyr Val Phe Trp Gly Gly Arg Glu
               180                 185                 190
   Gly Tyr Glu Thr Leu Leu Asn Thr Asp Leu Gly Leu Glu Leu Asp Asn
           195                 200                 205
   Met Ala Arg Leu Met Lys Met Ala Val Glu Tyr Gly Arg Ala Asn Gly
       210                 215                 220
   Phe Asp Gly Asp Phe Tyr Ile Glu Pro Lys Pro Lys Glu Pro Thr Lys
   225                 230                 235                 240
   His Gln Tyr Asp Phe Asp Thr Ala Thr Val Leu Ala Phe Leu Arg Lys
                   245                 250                 255
   Tyr Gly Leu Glu Lys Asp Phe Lys Met Asn Ile Glu Ala Asn His Ala
               260                 265                 270
   Thr Leu Ala Gly His Thr Phe Glu His Glu Leu Ala Met Ala Arg Val
           275                 280                 285
   Asn Gly Ala Phe Gly Ser Val Asp Ala Asn Gln Gly Asp Pro Asn Leu
       290                 295                 300
   Gly Trp Asp Thr Asp Gln Phe Pro Thr Asp Val His Ser Ala Thr Leu
   305                 310                 315                 320
   Ala Met Leu Glu Val Leu Lys Ala Gly Gly Phe Thr Asn Gly Gly Leu
                   325                 330                 335
   Asn Phe Asp Ala Lys Val Arg Arg Gly Ser Phe Glu Phe Asp Asp Ile
               340                 345                 350
   Ala Tyr Gly Tyr Ile Ala Gly Met Asp Thr Phe Ala Leu Gly Leu Ile
           355                 360                 365
   Lys Ala Ala Glu Ile Ile Asp Asp Gly Arg Ile Ala Lys Phe Val Asp
       370                 375                 380
   Asp Arg Tyr Ala Ser Tyr Lys Thr Gly Ile Gly Lys Ala Ile Val Asp
   385                 390                 395                 400
   Gly Thr Thr Ser Leu Glu Glu Leu Glu Gln Tyr Val Leu Thr His Ser
                   405                 410                 415
   Glu Pro Val Met Gln Ser Gly Arg Gln Glu Val Leu Glu Thr Ile Val
               420                 425                 430
   Asn Asn Ile Leu Phe Arg
           435